# EUROPÄISCHE PATENTSCHRIFT

(11) **EP 0 596 260 B1**
(45) Veröffentlichungstag und Bekanntmachung des Hinweises auf die Patenterteilung: **06.12.1995**
(21) Anmeldenummer: 93115952.9
(22) Anmeldetag: 02.10.1993
(51) Int. Cl.: A61M 1/36

(54) **Gerät zur medizinischen Ozontherapie durch Transfusion oder Infusion**
Device for medical ozontherapy by transfusion or infusion
Dispositif pour l'ozonthérapie médicale par transfusion ou perfusion

(30) Priorität: 05.11.1992 DE 9215078 U
(43) Veröffentlichungstag der Anmeldung: 11.05.1994
(73) Patentinhaber: CLINICO INFUSIONSTECHNIK GmbH, D-36251 Bad Hersfeld (DE)
(72) Erfinder: Heinzerling, Walter Chr., D-36251 Bad Hersfeld (DE); Schadt, Wolfgang, D-36199 Rotenburg (DE)
(74) Vertreter: von Raffay, Vincenz, Dipl.-Ing.

(56) Entgegenhaltungen:
- DE-A- 2 919 343
- DE-A- 3 232 716
- US-A- 2 879 784
- US-A- 3 042 038
- US-A- 4 269 222
- US-A- 5 015 369

## Beschreibung

Die Erfindung betrifft ein Gerät nach dem Oberbegriff des Anspruches 1.

Aus der DE-A1 32 32 716 ist ein Gerät nach dem Oberbegriff des Anspruches 1 bekannt. Dieses Gerät weist zwei parallele Leitungen auf, von denen die eine der Blutentnahme und die andere der Infusion bzw. Transfusion des mit Ozon angereicherten Blutes dient. In jeder Leitung ist ein Ventil angeordnet, das geöffnet oder geschlossen werden kann. Bei diesem Gerät handelt es sich um ein solches, das für eine "Dauerbehandlung" eingesetzt wird, und bei der beide Leitungen mit dem Patienten verbunden sind. Die Förderung des Blutes kann mit Hilfe einer Pumpe erfolgen.

Bekannte Geräte, die für eine Einmalbehandlung eingesetzt wurden, weisen lediglich eine Schlauchleitung auf, die sowohl der Blutentnahme als auch der Reinfusion dienen.

Nach neueren, wissenschaftlichen Erkenntnissen ist es erstrebenswert, die Reinfusion des ozonisierten Blutes oder die Infusion von ozonisierter Infusionsflüssigkeit unter schonenden Schwerkraftbedingungen durchzuführen. Insbesondere haben sich die Leukozyten bei der Ozon-Eigenblutbehandlung als besonders konzentrationsempfindlich erwiesen, so daß eine Reinfusion unter Schwerkraftbedingungen den Wirkungsgrad der Therapie wesentlich erhöht und gleichzeitig die Gefahr einer Hämolyse vermieden wird.

Grundsätzlich wurde bisher bei der sog. "Großen Eigenblutbehandlung" entweder ein Ein-Wege-System-Gerät sowohl zur Blutentnahme als auch zur Reinfusion eingesetzt oder es wurde ein aufwendiges System mit Pumpe und Kreislaufförderung zur Dauerbehandlung verwendet.

Der Erfindung liegt nun die Aufgabe zugrunde, ein Gerät der eingangs genannten Art zu schaffen, bei dem eine schonende Reinfusion unter Schwerkraft stattfindet, für einen guten Wirkungsgrad gesorgt ist und gleichzeitig eine hohe Betriebssicherheit unter Berücksichtigung hoher hygienischer Anforderungen sichergestellt sind.

Diese Aufgabe wird durch das Kennzeichen des Anspruches 1 gelöst.

Erfindungsgemäß weist das Gerät zwei parallele Schlauchleitungen auf, die die jeweils nur in einer Richtung durchströmt werden. Die Verbindung zur Reinfusion durch Schwerkraftwirkung erfolgt erst, wenn die Infusions- bzw. Transfusionsleitung entlüftet ist, d.h. wenn das Blut zur Reinfusion auch das Injektionsstück erreicht hat. Es wird auch sicher verhindert, daß in der Entnahmeleitung Blut verbleibt, das bei der Reinfusion nicht über den Filter geführt wird. Darüberhinaus ist das Gerät universell zur Transfusion aber auch zur Infusion mit Infusionsflüssigkeit einsetzbar. Das Ventil, das der Tropfkammer nachgeschaltet ist, verhindert sicher ein Leerlaufen der Tropfkammer. Es stellt auch einen Dichtverschluß gegen unbeabsichtigte Überdrücke im System stromaufwärts von der Tropfkammer dar. Somit ist auch hier die Möglichkeit einer Luftembolie ausgeschlossen. Bei der Reinfusion des vorher entnommenen Blutes wird das gesamte reinfundierte Blut über die Filterfläche in die Tropfkammer geleitet. Das evtl. in der Entnahmeleitung verbliebene Blut kann nicht reinfundiert werden, da dieses durch das Rückschlagventil sicher verhindert wird. Die gesamte Reinfusion findet unter Schwerkraftwirkung statt, wohingegen die Blutentnahme mit Hilfe des Unterdrucks in der Vakuumflasche sichergestellt ist.

In vorteilhafter Weise kann das Gerät so ausgebildet sein, wie in Anspruch 2 angegeben. Die Ausbildung des Ventilkörpers als schwimmender Auftriebskörper stellt sicher, daß der Ventilkörper beim Befüllen der Tropfkammer mit dem Flüssigkeitsspiegel nach oben bewegt wird, so daß sich das Ventil öffnet. Andererseits wird bei kritischem Absinken des Flüssigkeitsspiegels ein Leerlaufen verhindert.

Im einzelnen kann die Ausbildung des Gerätes so erfolgen, wie in den Ansprüchen 3 und 4 angegeben.

Es sind unterschiedliche Ausgestaltungen der Tropfkammer mit Filterelement und Ventilkörper möglich.

Die Ausbildung nach Anspruch 5 stellt eine besonders vorteilhafte Ausführungsform als Transfusionsgerät zur Eigenblutbehandlung dar. Entsprechendes gilt für die andere Ausführungsform nach Anspruch 6.

Die in Anspruch 7 definierte Ausführungsform stellt eine einfach aufgebaute Ausgestaltung als Infusionsgerät dar.

Im folgenden wird die Erfindung unter Hinweis auf die Zeichnung anhand verschiedener Ausführungsbeispiele näher erläutert.

Es zeigt:
- Fig. 1: eine prinzipielle Darstellung der wesentlichen Bauteile des Gerätes nach der Erfindung;
- Fig. 2: eine teilweise geschnittene Seitenansicht einer Tropfkammer mit Auslaufventil;
- Fig. 3: die Einzelheit X der Fig. 2 im vergrößerten Maßstab;
- Fig. 4: eine der Fig. 2 entsprechende Ansicht einer Tropfkammer, jedoch mit einem anderen Filter;
- Fig. 5: die Einzelheit Y der Fig. 4 im vergrößerten Maßstab;
- Fig. 6: einen Schnitt gemäß der Linie A-B der Fig. 5;
- Fig. 7: eine den Fig. 2 und 4 entsprechende Ansicht einer weiteren Ausführungsform, bei der Filter und Ventilkörper fest miteinander verbunden sind; und
- Fig. 8: einen Schnitt durch eine weitere Ausführungsform eines Ventils mit Auftriebskammer.

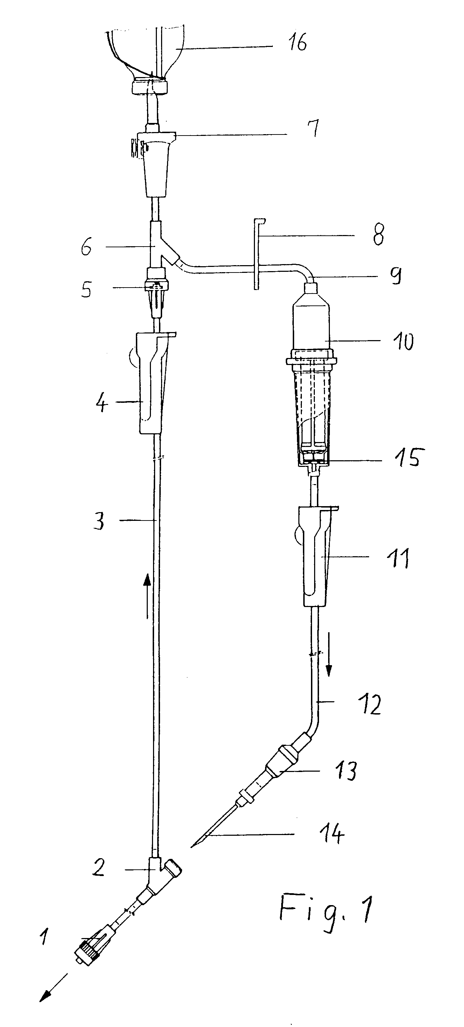

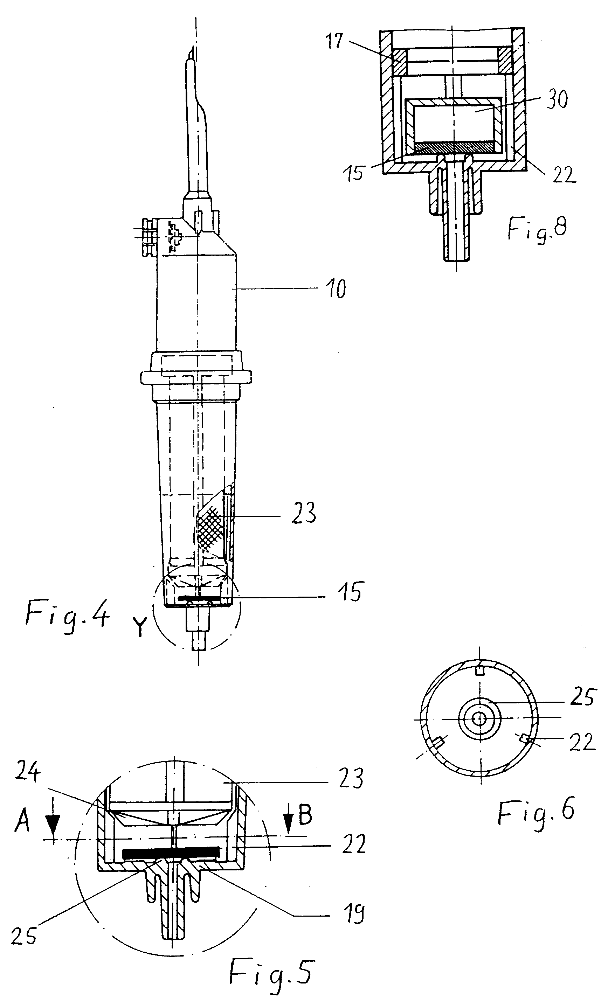

Das in Fig. 1 dargestellte Infusionsgerät besteht aus einem Konnektor 1, der der Verbindung mit dem zur Vene des Patienten führenden Besteck dient. Von einem Y-Stück 2 mit Einstichmembran führt eine Blutentnahmeleitung 3 über eine Rollenklemme 4 zu einem Rückschlagventil 5, das nur in Entnahmerichtung öffnet. Bei einer entgegengesetzten Strömung schließt das Rückschlagventil 5 sofort. Die Blutentnahmeleitung 3 ist über ein zweites Y-Stück 6 und eine Entnahmespike mit Belüftung und integriertem Rückschlagventil 7 mit einer Vakuumflasche 16 verbindbar. In der Vakuumflasche 16 erfolgt die Anreicherung mit Ozon in herkömmlicher Weise.

Parallel zu der Blutentnahmeleitung 3 verläuft eine Infusions- oder Transfusionsleitung 9, 12. Diese geht aus von dem Y-Stück 6 und ist mit dem Y-Stück 2 über ein Injektionsstück 13 und eine Verbindungskanüle 14 verbindbar. In der Leitung 9 sind in Strömungsrichtung nacheinander eine Schiebeklemme 8, eine Tropfkammer 10 mit Filter, ein Auslaufventil 15 und eine Rollklemme 11 angeordnet.

Zur Durchführung einer Therapie wird das Blut des Patienten über die Blutentnahmeleitung 3 entnommen und bei entsprechender Strömung in Entnahmerichtung ist das Rückschlagventil 5 offen. Wenn die gewünschte Menge an Blut in der Vakuumflasche 16 gesammelt wurde, erfolgt dort die Anreicherung mit Ozon. Die Reinfusion erfolgt über die Leitung 9,12. Das Rückschlagventil 5 verhindert sicher eine Strömung von Blut oder Luft durch die Entnahmeleitung 3 zum Patienten.

In der Tropfkammer 10 wird das gesamte, ozonisierte Blut gefiltert. Das Auslaufventil 15, dessen Ventilkörper in vorteilhafter Weise als Auftriebskörper ausgebildet ist, behindert die Strömung in Richtung des Patienten nicht, solange nicht die Gefahr eines Leerlaufens besteht, d.h. daß der Ventilkörper nicht mehr schwimmt und solange nicht ein unerwünschter Überdruck auftritt.

Das Auslaufventil kann in Verbindung mit dem Filter in unterschiedlicher Weise ausgebildet sein.

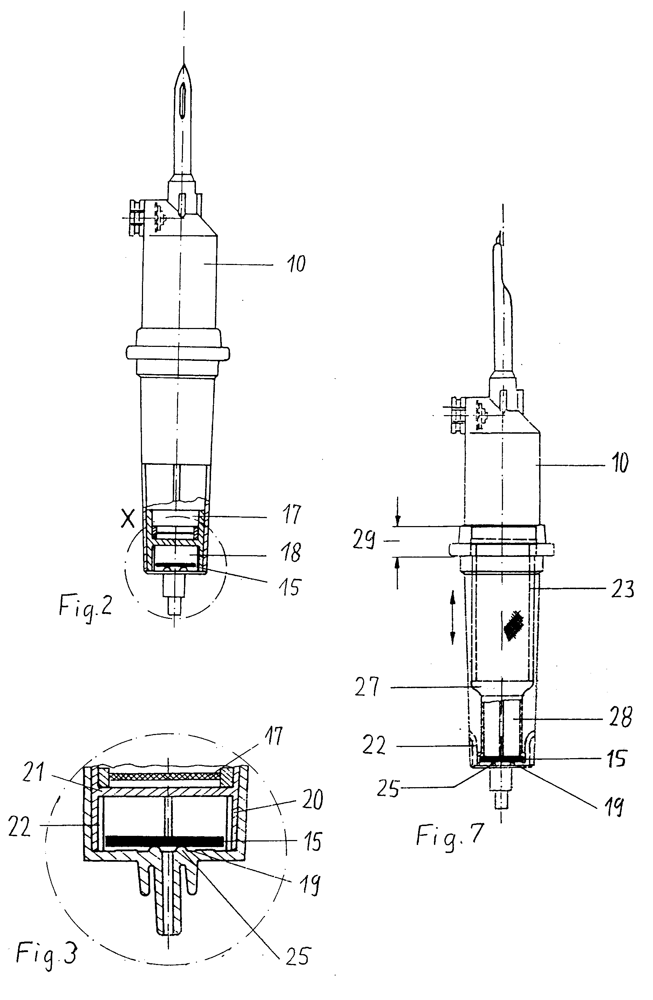

Bei der Ausführungsform nach den Fig. 2 und 3 handelt es sich um eine Tropfkammer mit einfach aufgebautem Scheibenfilter. Dieses Gerät wird im wesentlichen als Infusionsgerät eingesetzt. Unmittelbar oberhalb des Bodens 19 der Tropfkammer 10 (siehe Fig. 3) ist ein Käfig 20 mit einem sternförmigen Quersteg 21 angeordnet. Oberhalb des Quersteges 21 befindet sich der einfach aufgebaute Scheibenfilter 17. Unterhalb ist Raum 18 für den Ventilkörper des Auslaufventiles 15. Dieser liegt in Schließstellung, d.h. wenn er nicht auf einer Infusionsflüssigkeit schwimmt, auf dem Ventilsitz 25 auf, der durch einen entsprechenden Ringwulst gebildet wird. Distanzrippen 22 sorgen für die Führung des Ventilkörpers.

Bei der Ausführungsform nach den Fig. 4 bis 6 handelt es sich um eine Tropfkammer mit Blutfilter 23, d.h. um ein Gerät zur Durchführung einer Transfusion vorzugsweise mit Eigenblut. Der Filter 23 ist topfförmig ausgebildet. Unterhalb des Filterbodens 24 ist ein Raum 18 für den Ventilkörper vorgesehen. Grundsätzlich ist der Aufbau in diesem Bereich so, wie bei der Ausführungsform nach den Fig. 2 und 3, anders ausgebildet ist nur der Filter.

Bei der Ausführungsform nach Fig. 7 handelt es sich ebenfalls um eine Tropfkammer zur Bluttransfusion mit einem Filter 23, der so aufgebaut ist, wie derjenige bei der Ausführungsform nach den Fig. 4 bis 6. Am Boden 27 des Filters 23, der geschlossen ist, ist ein geschlossener Hohlraum 28 vorgesehen, der eine Auftriebskammer bildet. An der Unterseite des Hohlraumes 28 ist der Ventilkörper fest angeordnet. Der gesamte Filter 23 mit Hohlraum 28 (Auftriebskammer) und Ventilkörper ist auf- und abbewegbar, und zwar geführt in einem axialen Gleitbereich 29.

In Fig. 8 ist eine Ausführungsform, vorzugsweise für ein Infusionsgerät, dargestellt, bei der der Ventilkörper 15 einen Hohlraum 30 aufweist, der eine entsprechende Schwimmkammer bildet. Der eigentliche scheibenförmige Ventilkörper ist mit einem Paßsitz in eine entsprechende Öffnung an der Unterseite der Schwimmkammer eingesetzt. Wird die Tropfkammer, an derem unteren Ende das Ventil angeordnet ist, mit Infusionslösung gefüllt, hebt sich die Schwimmkammer mit dem scheibenförmigen Ventilkörper von der Auslauföffnung ab und gibt den Durchfluss frei. Bei Absinken des Flüssigkeitsspiegels wird die Auslauföffnung durch das Eigengewicht der Schwimmkammer zuverlässig verschlossen und so eine Luftinfusion verhindert. Auch bei dieser Ausführungsform sind Distanzrippen 22 vorgesehen, die die Lage des Ventilkörpers genau definieren und so eine einwandfreie Funktion sicherstellen.

Sämtlichen Ventilkörpern, ob nun als einfache Ventilscheibe ausgebildet oder als fest mit dem Filter verbunden, stellen sicher, daß ein Leerlaufen nicht stattfinden kann. Hierdurch ist es in vorteilhafter Weise auch möglich, daß für einen Infusionsbehälterwechsel (bei Entleerung) der normale Entlüftungsvorgang der Schlauchleitung eines Infusionsgerätes nicht erneut durchgeführt werden muß. Neben einer Zeitersparnis (neue Entlüftung) reduziert sich bei sachgemäßer Handhabung darüber hinaus das Kontaminationsrisiko durch Abkoppelung der Infusionsleitung.

Bei einer vorteilhaften Ausführungsform besteht der Ventilkörper aus einem thermoplastischen Elastomer, Silikon oder Silikonkautschuk, mit einer Dichte von 0,15 bis 0,9 kg/dm³. Bei der Ausführungsform nach Fig. 7 weist der gesamte Schwimmkörper, gebildet durch Filter, Auftriebskammer (Hohlraum 28), und Ventilkörper, eine Gesamtdichte in dem oben wiedergegebenen Bereich auf.

## Patentansprüche

1. Gerät zur medizinischen Ozontherapie durch Transfusion oder Infusion, mit einer vom Patienten zu einer Mischflasche führenden Blutentnahmeleitung (3) und einer zur Bildung eines Zwei-Wege-Systems parallel verlaufen den Infusions- bzw. Transfusionsleitung (9,12), wobei in der Blutentnahmeleitung ein in Entnahmerichtung öffnendes Ventil (5) angeordnet ist, dadurch gekennzeichnet, daß die Mischflasche (16) als Vakuumflasche ausgebildet ist,
daß das Ventil ein Rückschlagventil ist, daß die Infusions- bzw. Transfusionsleitung (9) über ein in Blutentnahmerichtung nach dem Rückschlagventil (5) in der Blutentnahmeleitung (3) angeordnetes Y-Stück (6) mit dieser verbunden ist, daß in der Infusions- bzw. Transfusionsleitung eine Tropfkammer (10) mit Filter (23) und Auslaufventil (15) angeordnet ist, und
daß die Infusions- bzw. Transfusionsleitung (12) über ein Injektionsstück (13) mit einem in Blutentnahmerichtung vor dem Rückschlagventil (5) in der Blutentnahmeleitung (3) angeordneten Y-Stück (2) mit Einstichmembran zur Reinfusion durch Schwerkraft verbindbar ist.

2. Gerät nach Anspruch 1, dadurch gekennzeichnet, daß der Ventilkörper des Auslaufventiles (15) als Auftriebskörper ausgebildet ist, der in der Infusionsflüssigkeit schwimmt.

3. Gerät nach Anspruch 1 oder 2, dadurch gekennzeichnet, daß der auf der Abgabeseite gelegene Ventilsitz durch einen Ringwulst (25) gebildet ist.

4. Gerät nach einem oder mehreren der vorstehenden Ansprüche, dadurch gekennzeichnet, daß der Ventilkörper (15) als Scheibe ausgebildet ist.

5. Gerät nach einem oder mehreren der vorstehenden Ansprüche 1 bis 3, dadurch gekennzeichnet, daß der Ventilkörper einen Hohlraum (30) aufweist, der als Auftriebskörper wirkt (Fig. 8).

6. Gerät nach einem oder mehreren der vorstehenden Ansprüche, dadurch gekennzeichnet, daß zur Führung des Ventilkörpers (15) Distanzrippen (22) an der Innenwand des Ventilgehäuses vorgesehen sind.

7. Gerät nach einem oder mehreren der vorstehenden Ansprüche, dadurch gekennzeichnet, daß der Filter (23) ein an sich bekannter topfförmiger Blutfilter, angeordnet in der Tropfkammer (10), ist (Fig. 4 bis 6).

8. Gerät nach Anspruch 7, dadurch gekennzeichnet, daß der Filter (23) auf- und abbewegbar in dem Tropfkammergehäuse angeordnet ist, und daß an seiner Unterseite der Ventilkörper fest angebracht ist, wobei der Filter einen eine Auftriebskammer bildenden Hohlraum (28) aufweist (Fig. 7).

9. Gerät nach einem oder mehreren der Ansprüche 1 bis 4, dadurch gekennzeichnet, daß der Filter als Scheibenfilter (17) ausgebildet ist (Fig. 2 und 3).

10. Gerät nach Anspruch 5, dadurch gekennzeichnet, daß der Ventilkörper (15) eine Dichtscheibe aufweist, die mit einem Paßsitz in eine entsprechende Öffnung an der Unterseite des die Auftriebskammer bildenden Hohlraumes (30) eingesetzt ist (Fig. 8).

## Claims

1. Apparatus for medical ozone therapy by transfusion or infusion, with a blood withdrawal line (3) leading from a patient to a mixing flask for forming a two-way system of parallel infusion or transfusion line (9, 12) and in the blood withdrawal line is located a valve (5) opening in the withdrawal direction, characterized in that the mixing flask (16) is constructed as a vacuum flask, that the valve is a check valve connecting the infusion or transfusion line (9) by means of a Y-piece (6) located in the blood withdrawal direction after the check valve (5) in the blood withdrawal line (3) to the said Y-piece (6), that in the infusion or transfusion line is located a drip chamber (10) with filter (23) and outlet valve (15) and that the infusion or transfusion line (12) is connectable by gravity by means of an injection piece (13) with a Y-piece (2) located in the blood withdrawal direction upstream of the check valve (5) in the blood withdrawal line (3) and having a puncture membrane for reinfusion.

2. Apparatus according to claim 1, characterized in that the valve body of the outlet valve (15) is constructed as a buoyancy member floating in the infusion fluid.

3. Apparatus according to claim 1 or 2, characterized in that the valve seat placed on the delivery side is formed by a torus (25).

4. Apparatus according to one or more of the preceding claims, characterized in that the valve body (15) is constructed as a disk.

5. Apparatus according to one or more of the preceding claims 1 to 3, characterized in that the valve body has a cavity (30), which acts as a buoyancy member (fig. 8).

6. Apparatus according to one or more of the preceding claims, characterized in that for guiding the valve body (15) spacing ribs (22) are provided on the inner wall of the valve housing.

7. Apparatus according to one or more of the preceding claims, characterized in that the filter (23) is a per se known cup-shaped filter located in the drip chamber (10) (figs. 4 to 6).

8. Apparatus according to claim 7, characterized in that the filter (23) is placed in the drip chamber housing so as to move up and down and that the valve body is fixed to its underside, the filter having a cavity (28) forming a buoyancy chamber (fig. 7).

9. Apparatus according to one or more of the claims 1 to 4, characterized in that the filter is constructed as a disk filter (17) (figs. 2 and 3).

10. Apparatus according to claim 5, characterized in that the valve body (15) has a sealing disk, which is inserted with press fit in a corresponding opening on the underside of the cavity (30) forming the buoyancy chamber (fig. 8).

## Revendications

1. Appareil pour la thérapie à l'ozone médicinale par transfusion ou perfusion, comportant une conduite (3) de prélèvement du sang qui va du patient à une bouteille de mélange et une conduite pour perfusion et transfusion (9, 12), orientée parallèlement, pour former un système à deux conduits, dans lequel, sur la conduite de prélèvement de sang, est disposé un clapet (5) qui s'ouvre dans le sens du prélèvement, caractérisé par le fait que la bouteille de mélange (16) est conçue en tant que bouteille sous vide,
que l'organe de fermeture est un clapet anti-retour, que la conduite (9) pour perfusion ou transfusion est reliée à la conduite (3) de prélèvement de sang par l'intermédiaire d'un raccord en Y (6) disposé sur la conduite (3) de prélèvement de sang, après le clapet anti-retour (5) dans le sens du prélèvement de sang,
que, sur la conduite pour perfusion ou transfusion, est disposé un flacon goutte à goutte (10) avec filtre (23) et clapet de sortie (15), et
par le fait que la conduite (12) pour perfusion ou transfusion peut être reliée, par l'intermédiaire d'un injecteur (13), avec un raccord en Y (2) à membrane pour piqûre qui est disposé sur la conduite (3) de prélèvement de sang, avant le clapet anti-retour (5) dans le sens du prélèvement de sang, pour réinjection sous l'action de la force de pesanteur.

2. Appareil selon la revendication 1, caractérisé par le fait que l'obturateur du clapet de sortie (15) est conçu en tant qu'élément à poussée ascensionnelle qui flotte dans le liquide pour perfusion.

3. Appareil selon la revendication 1 ou 2, caractérisé par le fait que le siège du clapet situé du côté sortie est formé d'un bourrelet annulaire (25).

4. Appareil selon l'une ou plusieurs des revendications précédentes, caractérisé par le fait que l'obturateur (15) a la forme d'un disque.

5. Appareil selon l'une ou plusieurs des revendications précédentes 1 à 3, caractérisé par le fait que l'obturateur présente un espace creux (30) qui agit comme élément à poussée ascensionnelle (figure 8).

6. Appareil selon l'une ou plusieurs des revendications précédentes, caractérisé par le fait que pour guider l'obturateur (15), des nervures réparties (22) sont prévues sur la paroi intérieure de l'enceinte du clapet.

7. Appareil selon l'une ou plusieurs des revendications précédentes, caractérisé par le fait que le filtre (23) est un filtre à sang, en forme de boisseau, connu en soi, disposé dans le flacon goutte à goutte (10) (figures 4 à 6).

8. Appareil selon la revendication 7, caractérisé par le fait que le filtre (23) est disposé dans l'enceinte du flacon goutte à goutte avec possibilité de monter et descendre et que l'obturateur est fixé à l'endroit de la face inférieure de ce boîtier, le filtre présentant un espace creux (28) qui forme une chambre de poussée ascensionnelle (figure 7).

9. Appareil selon l'une ou plusieurs des revendications 1 à 4, caractérisé par le fait que le filtre est conçu en tant que filtre à disque (17). (figures 2 et 3).

10. Appareil selon la revendication 5, caractérisé par le fait que l'obturateur (15) présente un disque d'étanchéité qui est inséré, à ajustement, dans une ouverture appropriée prévue sur la face inférieure de l'espace creux (30) formant la chambre de poussée ascensionnelle (figure 8).
